# EUROPEAN PATENT APPLICATION

(11) **EP 3 849 287 A2**
(43) Date of publication of application: **14.07.2021**
(21) Application number: 21150344.6
(22) Date of filing: 05.01.2021
(51) Int. Cl.: H05K 1/18, H05K 3/28, A61B 17/115, A61B 90/90, A61B 17/00, A61B 90/00, H05K 1/03

(54) **SURGICAL APPARATUS**

(30) Priority: 08.01.2020 US 202062958342 P; 11.12.2020 US 202017119755
(71) Applicant: Covidien LP, Mansfield, MA 02048 (US)
(72) Inventor: SGROI, Anthony, Wallingford, Connecticut 06492 (US); MOZDZIERZ, Patrick, Glastonbury, Connecticut 06033 (US)
(74) Representative: Maschio, Antonio

(57) **Abstract**

A powered surgical device includes a wire harness having a flex cable. The flex cable possesses cover layers which increase the number of disinfection and sterilization cycles that the wire harness/flex cable may be subjected to, thereby increasing the life of the powered surgical device.

## Description

### CROSS-REFERENCE TO RELATED APPLICATIONS

This application claims the benefit of and priority to U.S. Provisional Patent Application Serial No. 62/958,342 filed January 8, 2020, the entire disclosure of which is incorporated by reference herein.

### BACKGROUND

### 1. Technical Field

This disclosure is directed to a wire harness for a powered surgical device. More specifically, this disclosure is related to wire harnesses having a flex cable possessing cover layers which increases the number of disinfection and sterilization cycles that the wire harness/flex cable may be subjected to, thereby increasing the life of the device.

### 2. Background

Surgical devices, e.g., surgical stapling devices, include an actuator or handle assembly, an elongated body portion or adapter assembly, and a reload including a tool assembly. The adapter assembly is supported on and extends distally from the actuator assembly and the reload is supported on a distal portion of the adapter assembly.

In known electrically powered surgical stapling devices, the actuator assembly supports a power supply, e.g., a battery pack that supplies power to a motor within the actuator assembly to drive different assemblies of the stapling device, e.g., an approximation assembly, a firing assembly, and a knife assembly. In some devices, a wire harness is coupled between the actuator assembly and the reload to facilitate communication between a chip in the reload and a processor in the actuator assembly. The chip in the reload may be provided to identify the type and/or size of the reload and/or whether the reload has been previously fired. This information is sent via the wire harness to the processor in the actuator assembly to determine whether the actuator and the reload are compatible, and/or whether the reload has not been used.

For reusable devices, the surgical device is subjected to disinfection and/or sterilization treatments, sometimes referred to as cycles, prior to reuse.

Improved surgical devices, capable of withstanding numerous disinfection and sterilization cycles, thereby increasing the useful life of the surgical devices, remain desirable.

### SUMMARY

One aspect of this disclosure is directed to a surgical device including an actuator assembly, an adapter assembly, and a tool assembly forming part of a reload assembly. The adapter assembly includes a wire harness having a proximal connector supported adjacent the actuator assembly and a distal connector supported adjacent the reload assembly. The wire harness possesses a flex cable with a cover layer on at least a portion of the flex cable.

A surgical device of the present disclosure can include an actuator assembly; an adapter assembly including an elongated body portion, the elongated body portion being releasably coupled to the actuator assembly; and a tool assembly supported on a distal portion of the elongated body portion, the tool assembly forming part of a reload assembly being releasably coupled to the distal portion of the elongated body portion. The adapter assembly includes a wire harness having a proximal connector supported adjacent the actuator assembly and a distal connector supported adjacent the reload assembly, the wire harness possessing a flex cable with a cover layer on at least a portion of the flex cable, the cover layer having at least a sacrificial layer bonded to a portion of the cover layer.

The cover layer can be bonded to the flex cable using an adhesive, heat, or combinations thereof.

The cover layer can be formed of a material selected from liquid crystal polymers, polyimides, polyetherimides, polyesters, or combinations thereof.

The liquid crystal polymers can be co-polymers of hydroxybenzoic acid (ester) and hydroxy naphthoic acid (ester).

The sacrificial layer can be formed of a material selected from liquid crystal polymers, polyimides, polyetherimides, polyesters, or combinations thereof.

The wire harness can include a service loop.

The present disclosure also provides an adapter assembly including an elongated body portion including a wire harness, the wire harness having a proximal connector, a distal connector, and a flex cable, the flex cable joining the proximal and distal connectors. The flex cable includes a cover layer on at least a portion of the flex cable, the cover layer having at least a sacrificial layer bonded to a portion of the cover layer.

The cover layer can be bonded to the flex cable using an adhesive, heat, or combinations thereof.

The cover layer can be formed of a material selected from liquid crystal polymers, polyimides, polyetherimides, polyesters, or combinations thereof.

The liquid crystal polymers are co-polymers of hydroxybenzoic acid (ester) and hydroxy naphthoic acid (ester).

The sacrificial layer can be formed of a material selected from liquid crystal polymers, polyimides, polyetherimides, polyesters, or combinations thereof.

The wire harness can include a service loop.

The present disclosure also provides a flex cable including a base layer having a top and bottom surface, the top surface having a top trace and a top solder pad and the bottom surface having a bottom trace and a bottom solder pad. The flex cable also includes a top cover layer bonded to the top surface covering the top trace and a portion of the top solder pad; a top sacrificial layer bonded to the top cover layer; a bottom cover layer bonded to the bottom surface covering the bottom trace and a portion of the bottom solder pad; and a bottom sacrificial layer bonded to the bottom cover layer.

The base layer of the flex cable can be a film.

The top cover layer can be bonded to the top surface covering the top trace and a portion of the top solder pad using an adhesive, heat, or combinations thereof.

The bottom cover layer can be bonded to the bottom surface covering the bottom trace and a portion of the bottom solder pad using an adhesive, heat, or combinations thereof.

The top cover layer, the bottom cover layer, or both, can be formed of a material selected from liquid crystal polymers, polyimides, polyetherimides, polyesters, or combinations thereof.

The liquid crystal polymers can be co-polymers of hydroxybenzoic acid (ester) and hydroxy naphthoic acid (ester).

The top sacrificial layer, the bottom sacrificial layer, or both, can be formed of a material selected from liquid crystal polymers, polyimides, polyetherimides, polyesters, or combinations thereof.

### BRIEF DESCRIPTION OF THE DRAWINGS

Various aspects of a powered surgical stapling device including a wire harness assembly with the disclosed flex cable are described herein below with reference to the drawings, wherein:
FIG. 1 is a side perspective view of exemplary aspects of the disclosed powered surgical stapling device;
FIG. 2 is a side perspective view of an adapter assembly of the powered surgical stapling device shown in FIG. 1 with a wire harness of the adapter assembly including a flex cable shown in phantom;
FIG. 3 is a side view of the wire harness of the adapter assembly of FIG. 2 including the flex cable;
FIG. 4 illustrates a top view of a single sided flex cable material including a base material with a bonded copper clad material along the top side;
FIG. 5 is a side view of the single sided flex cable material of FIG. 4;
FIG. 6 is a view of the single sided flex cable material of FIG. 4 with a portion of the copper clad etched away and includes the cut-line of a flex cable;
FIG. 7 illustrates a partially fabricated flex cable cut along the cut-line shown in FIG. 6;
FIG. 8 is a side view of the partially fabricated flex cable of FIG. 7;
FIG. 9 illustrates a completed flex cable resulting in the bonding of a cover layer to the partial fabrication of FIGS. 7 and 8;
FIG. 10 is a side view of the completed flex cable of FIG. 9;
FIG. 11 illustrates a side view of a doubled sided flex cable material having a base material with a bonded copper clad material integrated on each side;
FIG. 12 illustrates a completed flex cable constructed using the doubled sided flex cable material of FIGS. 10 and 11 with a cover layer bonded along each side; and
FIG. 13 illustrates the flex cable of FIG. 12 with a sacrificial layer of cover layer material bonded along each side.

### DETAILED DESCRIPTION

The disclosed surgical device will now be described in detail with reference to the drawings in which like reference numerals designate identical or corresponding elements in each of the several views. In this description, the term "proximal" is used generally to refer to that portion of the device that is closer to a clinician, while the term "distal" is used generally to refer to that portion of the device that is farther from the clinician. In addition, the term clinician is used generally to refer to medical personnel including doctors, nurses, and support personnel.

The disclosed wire harness forms part of a surgical device that includes an actuator assembly including a handle, an adapter assembly extending distally from the actuator assembly, and a tool or reload assembly supported on a distal portion of the adapter assembly. The adapter assembly includes the wire harness, which extends between the actuator assembly and the reload assembly to facilitate communication between the actuator assembly and the reload assembly. The wire harness possesses a flex cable with a cover on at least a portion thereof. The cover on the flex cable increases the durability of the flex cable, thereby increasing the number of disinfection and sterilization cycles that the flex cable can be subjected to. This, in turn, increases the longevity of the flex cable and the device including same.

Although the disclosure is directed to a circular stapling device, it is envisioned that the disclosed wire assembly could be incorporated into a variety of different devices including linear stapling devices, ligation devices, clip appliers, and vessel sealing devices.

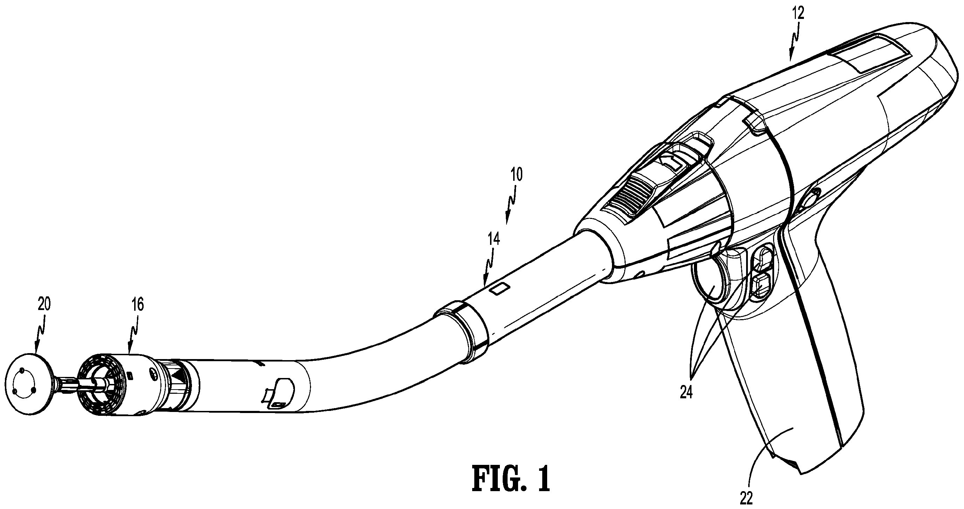

FIG. 1 illustrates a surgical stapling device 10 including an actuator assembly 12, an adapter assembly 14, and a reload assembly 16. The adapter assembly 14 has a proximal portion that is releasably coupled to the actuator assembly 12, and a distal portion that is releasably coupled to the reload assembly 16. The surgical stapling device 10 is an electromechanically powered system such as disclosed in U.S. Patent Publication Nos. 2015/0108201, 2015/0048140, and 2015/0076206. The actuator assembly 12 includes a stationary handle 22 and a plurality of actuation buttons 24 that control different functions of the stapling device 10, e.g., approximation, stapling, and cutting. In aspects of the disclosure, the stationary handle 22 supports a battery pack (not shown) for powering the actuator assembly 12 and a processor (not shown) for controlling operation of the actuator assembly 12.

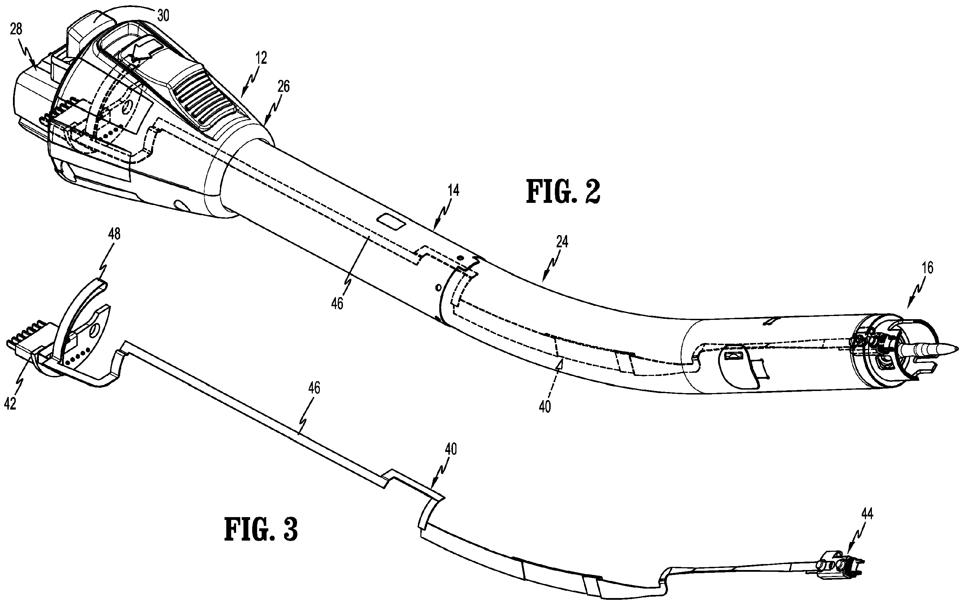

FIGS. 2 and 3 illustrate the adapter assembly 14 positioned between the actuator assembly 12 and the reload assembly 16 to translate power from the actuator assembly 12 to the reload assembly 16. The adapter assembly 14 includes an elongated body 24, a rotation assembly 26, and a coupling assembly 28. The coupling assembly 28 includes a release button 30 that can be depressed to uncouple the adapter assembly 26 from the actuator assembly 12.

Referring also to FIGS. 2 and 3, the adapter assembly 14 includes a wire harness 40 that includes a proximal connector 42, a distal connector 44, a flex cable 46, and a service loop 48. The proximal connector 42 is connected to the actuator assembly 12 and the distal connector 44 is connected to the reload assembly 16. In aspects of the disclosure, the actuator assembly 12 includes a processor (not shown) and the reload assembly 16 includes a chip (not shown). The wire harness 40 provides a path of communication between the processor (not shown) and the chip (not shown) to allow information stored in the chip to be sent to the processor. In aspects of the disclosure, the chip includes information such as the type of the reload 16, the size of the reload, and/or the status of the reload, e.g., fired or unfired.

The service loop 48 includes an overlapping portion of flex cable 46 which is fed out as the connectors 42 and 44 become further spaced to allow for some degree of relative rotation between the proximal and distal connectors 42, 44 to occur without the proximal and/or distal connectors 42, 44 becoming separated from the actuator assembly 12 and reload assembly 16, respectively.

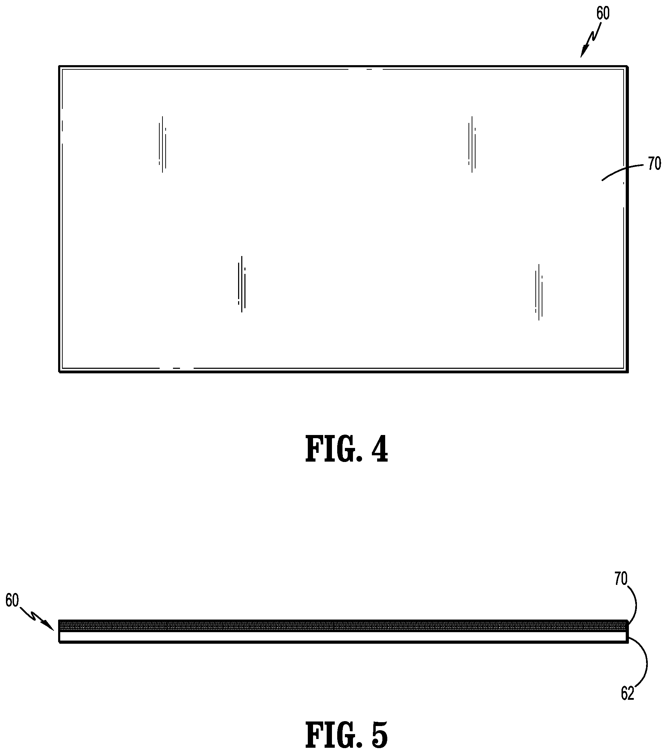

Flex cable 46 can be constructed from any know material. In one embodiment, for example, flex cable 46 is constructed from base materials such as PYRALUX® brand sold by the DuPont™ company. Such materials come in a variety of forms such as single and double copper-clad layers. As seen in FIGS. 4 and 5, a sheet of flex cable material 60 is shown having a single copper-clad layer 70. The flex cable material 60 includes a base layer 62 (i.e., a film, which may be formed of a suitable material such as a polyimide) with the copper-clad layer 70 bonded along the entire surface of the base layer 62. From this, the flex cable 46 can be fabricated.

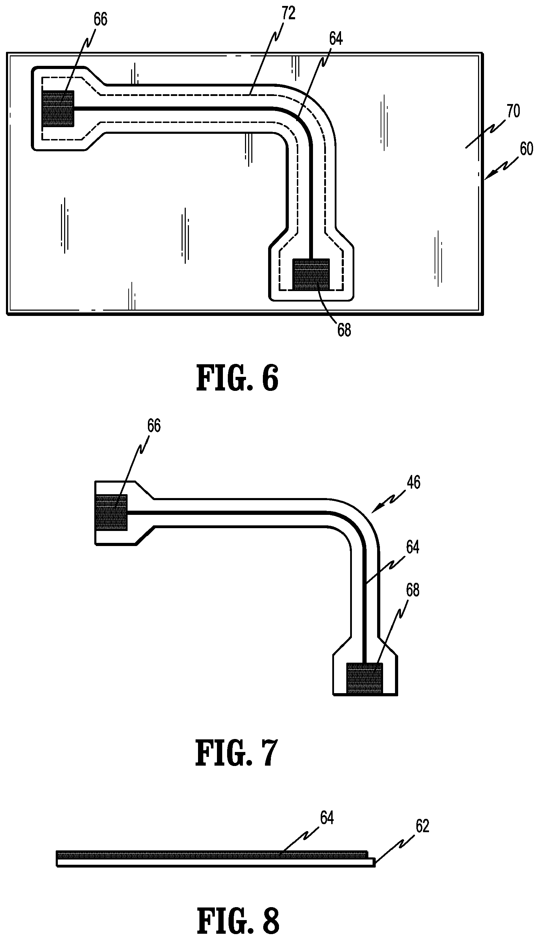

Simple fabrication of the flex cable 46, and by way of example only, is described with reference to FIGS. 5 through 9. Fabrication of the flex cable 46 begins with etching away the copper-clad layer 70 of the base layer 62 to create a wire trace 64 and solder pads 66, 68. As seen in FIG. 6, the copper-clad layer 70 has been etched away and all that remains is the wire trace 64 and the pair of solder pads 66, 68. Dashed line 72 (FIG. 6) represents a cut line that will allow for the flex cable 46 to be removed from the flex cable material 60. As seen in FIG. 7, the completed flex cable 46 is cut away (laser or die-cut) and removed from the base layer 62. As seen in FIGS. 7 and 8, the resulting flex cable 46 has exposed wire trace 64 and solder pads 66, 68 along the top, and the base layer 62 along the bottom, where the base layer 62 is a dielectric. Fabrication of the flex cable 46 is not yet complete in FIGS. 7 and 8, as the wire trace 64 is exposed.

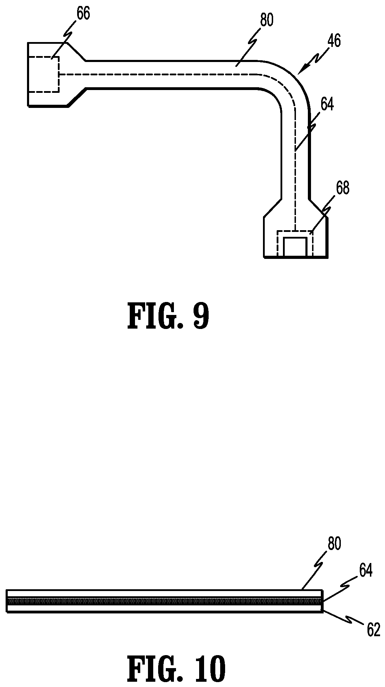

FIGS. 9 and 10 illustrate a cover layer 80 applied to cover or protect the wire trace 64 as well as cover a perimeter portion of the solder pads 66, 68. The cover layer 80 includes the same shape and can be bonded to the flex cable 46. Once completed, only a portion of the solder pads 66, 68 are exposed to allow for soldering to a mating component(s) (not shown).

Bonding of the cover layer 80 to the flex cable 46 can be achieved by a variety of methods. In one example, the cover layer 80 can be bonded to the flex cable 46 utilizing an adhesive. In another example, the cover layer 80 can be bonded to the flex cable 46 using heat, causing the layers to melt together. Other methods are possible.

Suitable materials for forming the cover layer 80 include, but are not limited to, liquid crystal polymers (LCP), polyimides, polyetherimides (including those commercially available as ULTEM®), polyesters, and the like, and any combinations thereof, as those skilled in the art can appreciate.

Liquid crystal polymers which may be used in forming the cover layer 80 include polymers of an aromatic or aliphatic dihydroxy compound, a polymer of an aromatic or aliphatic dicarboxylic acid, a polymer of an aromatic hydroxycarboxylic acid, a polymer of an aromatic diamine, aromatic hydroxyamine, aromatic amino carboxylic acid, etc., and the like.

Exemplary liquid crystal polymers include those commercially available under the trade name VECSTAR™ supplied by Kuraray Co., Ltd., and BIAC™ from W.L. Gore & Associates, Inc. VECSTAR™ is a co-polymer of hydroxybenzoic acid (ester) and hydroxy naphthoic acid (ester).

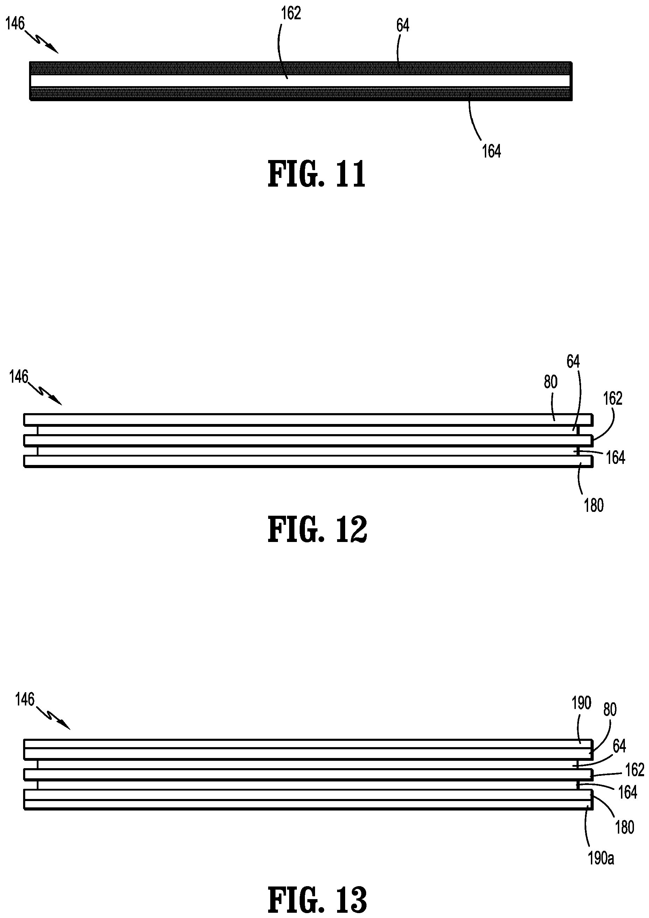

In addition to the flex cable 46 described above, a flex cable 146 using double copper-clad layers 164, 164a is described. Such an exemplary flex cable 146 is described with reference to FIG. 11. The flex cable 146 can be fabricated including a second wire trace (sometimes referred to as a bottom trace) 164 and a second pair of solder pads (sometimes referred to as bottom solder pads) 166, 168 along the bottom surface. The addition of a bottom cover layer 180 along the bottom portion creates a flex cable having a double-sided set of wire traces 64, 164 (sometimes referred to herein as a top trace and a bottom trace, respectively), and solder pads 66, 68, 166, 168 (sometimes referred to herein as top solder pads and bottom solder pads, respectively). This is illustrated in FIG. 12.

FIG. 13 illustrates the flex cable 146 of FIG. 12 is shown including additional layers of cover layer materials denoted as sacrificial layers 190, 190a. The sacrificial layers 190, 190a allow for numerous options including, for example,
- The sacrificial layers 190, 190a can include a thick layer of low-cost material, allowing for numerous disinfection and sterilization cycles that are necessary to break down the sacrificial layers 190, 190a. This extends the life of the flex cable 146 in proportion to the thickness of the sacrificial layers 190, 190a.
- The sacrificial layers 190, 190a can include a very thin layer of LCP film allowing for near unlimited disinfection and sterilization cycles. Since the LCP sacrificial layers 190, 190a are very thin, material costs can be lowered.
- The sacrificial layers 190, 190a can include both a thick layer of low-cost material with a complete LCP flex cable 146 as per FIG. 12. In this example, the complete LCP flex cable 146 as per FIG. 12 is fabricated using very thin layers of LCP film resisting numerous disinfection and sterilization cycles. Using very thin LCP film thicknesses may reduce the strength of the flex cable, so the addition of a low cost and thicker set of sacrificial layers can provide the needed strength of the cable for enhanced handling.

Suitable materials for forming the sacrificial layer include, but are not limited to, liquid crystal polymers, low-cost materials such as polyimides, polyetherimides (including those commercially available as ULTEM®), polyesters, and the like, and any combinations thereof. In aspects of the disclosure, as LCP films can be expensive, it may be desirable to form sacrificial layers 190, 190a with materials lower in cost than LCP.

Bonding of the sacrificial layer(s) to the cover layer(s) can be achieved by a variety of methods, including the use of adhesives, heat (causing the layers to melt together) or combinations thereof. Other methods are possible.

Of course, it is contemplated by this disclosure that flex cable 146 can have additive sacrificial layers bonded thereto. For example, flex cable 146 can have a second sacrificial layer of polyimide bonded along each side of flex cable 146.

In use, the surgical device of the disclosure is reusable, so that the cost of numerous procedures can be spread across the life of the reusable device resulting in a lower cost per procedure. Once firing of the surgical device of the disclosure is complete, the reusable device is disinfected using an autowash process. Such processes can include a high pH solution (potassium hydroxide) which can be harsh on sensitive electronic components and flex cables. After disinfection is complete, sterilization may be performed using an autoclave process in which the device is subjected to high temperature and high-pressure steam. The surgical device of the disclosure, possessing the cover layers on the flex cables as described above, can withstand the harsh environments of common disinfection and sterilization processes.

Persons skilled in the art will understand that the devices and methods specifically described herein and illustrated in the accompanying drawings are non-limiting exemplary aspects of the disclosure. It is envisioned that the elements and features illustrated or described in connection with one exemplary aspect of the disclosure may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:
1. A surgical device comprising: an actuator assembly; an adapter assembly including an elongated body portion, the elongated body portion being releasably coupled to the actuator assembly; and a tool assembly supported on a distal portion of the elongated body portion, the tool assembly forming part of a reload assembly being releasably coupled to the distal portion of the elongated body portion; wherein the adapter assembly includes a wire harness having a proximal connector supported adjacent the actuator assembly and a distal connector supported adjacent the reload assembly, the wire harness possessing a flex cable with a cover layer on at least a portion of the flex cable, the cover layer having at least a sacrificial layer bonded to a portion of the cover layer.
2. The surgical device of paragraph 1, wherein the cover layer is bonded to the flex cable using an adhesive, heat, or combinations thereof.
3. The surgical device of paragraph 1, wherein the cover layer is formed of a material selected from liquid crystal polymers, polyimides, polyetherimides, polyesters, or combinations thereof.
4. The surgical device of paragraph 3, wherein the liquid crystal polymers are co-polymers of hydroxybenzoic acid (ester) and hydroxy naphthoic acid (ester).
5. The surgical device of paragraph 1, wherein the sacrificial layer is formed of a material selected from liquid crystal polymers, polyimides, polyetherimides, polyesters, or combinations thereof.
6. The surgical device of paragraph 1, wherein the wire harness includes a service loop.
7. An adapter assembly comprising: an elongated body portion including a wire harness, the wire harness having a proximal connector, a distal connector, and a flex cable, the flex cable joining the proximal and distal connectors, the flex cable including a cover layer on at least a portion of the flex cable, the cover layer having at least a sacrificial layer bonded to a portion of the cover layer.
8. The adapter assembly of paragraph 7, wherein the cover layer is bonded to the flex cable using an adhesive, heat, or combinations thereof.
9. The adapter assembly of paragraph 7, wherein the cover layer is formed of a material selected from liquid crystal polymers, polyimides, polyetherimides, polyesters, or combinations thereof.
10. The adapter assembly of paragraph 9, wherein the liquid crystal polymers are co-polymers of hydroxybenzoic acid (ester) and hydroxy naphthoic acid (ester).
11. The adapter assembly of paragraph 7, wherein the sacrificial layer is formed of a material selected from liquid crystal polymers, polyimides, polyetherimides, polyesters, or combinations thereof.
12. The adapter assembly of paragraph 7, wherein the wire harness includes a service loop.
13. A flex cable comprising: a base layer having a top and bottom surface, the top surface having a top trace and a top solder pad and the bottom surface having a bottom trace and a bottom solder pad; a top cover layer bonded to the top surface covering the top trace and a portion of the top solder pad; a top sacrificial layer bonded to the top cover layer; a bottom cover layer bonded to the bottom surface covering the bottom trace and a portion of the bottom solder pad; and a bottom sacrificial layer bonded to the bottom cover layer.
14. The flex cable of paragraph 13, wherein the base layer is a film.
15. The flex cable of paragraph 13, wherein the top cover layer is bonded to the top surface covering the top trace and a portion of the top solder pad using an adhesive, heat, or combinations thereof.
16. The flex cable of paragraph 13, wherein the bottom cover layer is bonded to the bottom surface covering the bottom trace and a portion of the bottom solder pad using an adhesive, heat, or combinations thereof.
17. The flex cable of paragraph 13, wherein the top cover layer, the bottom cover layer, or both, are formed of a material selected from liquid crystal polymers, polyimides, polyetherimides, polyesters, or combinations thereof.
18. The flex cable of paragraph 17, wherein the liquid crystal polymers are co-polymers of hydroxybenzoic acid (ester) and hydroxy naphthoic acid (ester).
19. The flex cable of paragraph 13, wherein the top sacrificial layer, the bottom sacrificial layer, or both, are formed of a material selected from liquid crystal polymers, polyimides, polyetherimides, polyesters, or combinations thereof.

## Claims

1. A surgical device comprising:
an actuator assembly;
an adapter assembly including an elongated body portion, the elongated body portion being releasably coupled to the actuator assembly; and
a tool assembly supported on a distal portion of the elongated body portion, the tool assembly forming part of a reload assembly being releasably coupled to the distal portion of the elongated body portion;
wherein the adapter assembly includes a wire harness having a proximal connector supported adjacent the actuator assembly and a distal connector supported adjacent the reload assembly, the wire harness possessing a flex cable with a cover layer on at least a portion of the flex cable, the cover layer having at least a sacrificial layer bonded to a portion of the cover layer.

2. The surgical device of claim 1, wherein the cover layer is bonded to the flex cable using an adhesive, heat, or combinations thereof.

3. The surgical device of claim 1 or claim 2, wherein the cover layer is formed of a material selected from liquid crystal polymers, polyimides, polyetherimides, polyesters, or combinations thereof.

4. The surgical device of claim 3, wherein the liquid crystal polymers are co-polymers of hydroxybenzoic acid (ester) and hydroxy naphthoic acid (ester).

5. The surgical device of any preceding claim, wherein the sacrificial layer is formed of a material selected from liquid crystal polymers, polyimides, polyetherimides, polyesters, or combinations thereof and/or wherein the wire harness includes a service loop.

6. An adapter assembly comprising:
an elongated body portion including a wire harness, the wire harness having a proximal connector, a distal connector, and a flex cable, the flex cable joining the proximal and distal connectors, the flex cable including a cover layer on at least a portion of the flex cable, the cover layer having at least a sacrificial layer bonded to a portion of the cover layer.

7. The adapter assembly of claim 6, wherein the cover layer is bonded to the flex cable using an adhesive, heat, or combinations thereof;and/or wherein the cover layer is formed of a material selected from liquid crystal polymers, polyimides, polyetherimides, polyesters, or combinations thereof.

8. The adapter assembly of claim 6 or claim 7, wherein the liquid crystal polymers are co-polymers of hydroxybenzoic acid (ester) and hydroxy naphthoic acid (ester); and/or wherein the sacrificial layer is formed of a material selected from liquid crystal polymers, polyimides, polyetherimides, polyesters, or combinations thereof.

9. The adapter assembly of any of claims 6 to 8, wherein the wire harness includes a service loop.

10. A flex cable comprising:
a base layer having a top and bottom surface, the top surface having a top trace and a top solder pad and the bottom surface having a bottom trace and a bottom solder pad;
a top cover layer bonded to the top surface covering the top trace and a portion of the top solder pad;
a top sacrificial layer bonded to the top cover layer;
a bottom cover layer bonded to the bottom surface covering the bottom trace and a portion of the bottom solder pad; and
a bottom sacrificial layer bonded to the bottom cover layer.

11. The flex cable of claim 10, wherein the base layer is a film; and/or wherein the top cover layer is bonded to the top surface covering the top trace and a portion of the top solder pad using an adhesive, heat, or combinations thereof.

12. The flex cable of claim 10 or claim 11, wherein the bottom cover layer is bonded to the bottom surface covering the bottom trace and a portion of the bottom solder pad using an adhesive, heat, or combinations thereof.

13. The flex cable of any of claims 10 to 12, wherein the top cover layer, the bottom cover layer, or both, are formed of a material selected from liquid crystal polymers, polyimides, polyetherimides, polyesters, or combinations thereof.

14. The flex cable of claim 13, wherein the liquid crystal polymers are co-polymers of hydroxybenzoic acid (ester) and hydroxy naphthoic acid (ester).

15. The flex cable of any of claims 10 to 14, wherein the top sacrificial layer, the bottom sacrificial layer, or both, are formed of a material selected from liquid crystal polymers, polyimides, polyetherimides, polyesters, or combinations thereof.
